# EUROPEAN PATENT SPECIFICATION

(11) **EP 2 442 755 B1**
(45) Date of publication and mention of the grant of the patent: **20.04.2016**
(21) Application number: 10730543.5
(22) Date of filing: 16.06.2010
(51) Int. Cl.: A61F 2/30, A61F 2/40

(54) **SET OF RECONSTRUCTION OF A FRACTURED SHOULDER JOINT**
REKONSTRUKTIONSSET FÜR EIN GEBROCHENES SCHULTERGELENK
ENSEMBLE DE RECONSTRUCTION D'UNE ARTICULATION D'EPAULE FRACTUREE

(30) Priority: 18.06.2009 FR 0954103
(43) Date of publication of application: 25.04.2012
(73) Proprietor: FX Solutions, 01000 Bourg en Bresse (FR)
(72) Inventor: LASCAR, Tristan, 06320 Cap D'ail (FR); OBERT, Laurent, 25320 Vorges les Pins (FR); MARTIN, Jean-Jacques, 01000 Bourg En Bresse (FR)
(74) Representative: Jeannet, Olivier
(86) International application number: PCT/IB2010/052698
(87) International publication number: WO 2010/146545

(56) References cited:
- WO-A-99/37254
- WO-A2-2007/082925
- FR-A1- 2 726 994
- FR-A1- 2 909 858
- US-A1- 2001 011 193

## Description

This invention relates to a set of reconstruction of a fractured shoulder joint.

A fracture of the shoulder joint often involves a fracture of one or two tuberosities of the upper end of the humerus, that is to say, the lesser tuberosity and the greater tuberosity. When treating this fracture, it is necessary to reposition perfectly any such tuberosities in place on the humerus, failing which the recovery of the joint function would be imperfect. In particular, if the tuberosities are not perfectly repositioned on the humerus these may hinder, even limit the joint movement.

For this reinstallation of tuberosities, a known shoulder prosthesis comprises a medullar stem equipped with an external fin pierced by one or more holes, this or these holes being intended for receiving one or more threads; after implementation of the medullar stem in the medullary canal of the humerus, this or these threads are able to form retention loops of the tuberosities on the medullar stem.

This technique is not fully satisfactory in terms of bone consolidation and of repositioning of the tuberosities. In fact, the fractured tuberosities may have an insufficient quantity of cancellous bone to achieve perfect bone consolidation, this quantity of cancellous bone decreasing with the age of the patient. In addition, the cortex of the tuberosities is often placed against the metal of the said fin or of the humeral stem, preventing or limiting generally, as a consequence, the bone consolidation between the tuberosities. The repositioning of the tuberosities is in turn relatively empirical, and the said fin and loop or loops may be insufficient to ensure the perfect immobilisation of the tuberosities in relation to the rest of the humerus. In addition, the fin does not prevent effectively the risk of displacement of the tuberosities towards the inner side of the joint.

The purpose of this invention is to provide a set of reconstruction for a fractured shoulder joint by overcoming the drawbacks mentioned above.

Document No. FR 2 726 994 describes an osteo-permeable cage for a shoulder prosthesis, placed at the end of a medullar stem and interposed between this medullar stem and the humeral head of the joint. On the side of the outer periphery of the humeral implant, the cage has a large opening, and repositioning in place of the tuberosities is performed by one or more cerclages.

The cage according to this prior document improves the possibility of bone consolidation of the tuberosities through the graft which it is able of comprising, but does not allow overcoming the other drawbacks mentioned above.

Document No. WO 99/37254 describes a shoulder prosthesis equipped with a median lateral "quiver", thin and hollow, for receiving bone chips or bone shavings or similar. This "quiver" also improves the possibility of bone consolidation of the tuberosities but, being comparable to a fin, it does not allow overcoming the other drawbacks mentioned above.

Document No. EP 1 415 621 describes a shoulder prosthesis comprising, in relation to the tuberosities, a massive smooth walled rounded boss. Below the tuberosities, this prosthesis comprises a sleeve adapted to be engaged on the medullar stem of the prosthesis, located, after installation into position, in the metaphyseal-diaphyseal area of this medullar stem. This sleeve delimits a housing with the latter.

This prosthesis improves the possibility of anchoring the medullar stem to the bone by growth of bone cell in the said housing but, however it does not allow overcoming the disadvantages mentioned above.

The document FR 2 909 858 discloses a set of reconstruction of a fractured shoulder joint according to the preamble of the appended main claim, in which the element of reconstruction is formed by a block of synthetic material or of osseous material.

The main objective of this invention is to provide a set of reconstruction allowing repositioning the tuberosities in relation to the shoulder prosthesis such that the bone consolidation occurs under optimum conditions.

Another objective of the invention is to provide such a set of reconstruction, allowing preventing effectively the risk of displacement of the tuberosities towards the inner side of the joint.

An additional objective of the invention is to provide a set of reconstruction the position of which in relation to the humeral implant is adaptable based on the configuration of the fracture.

Yet another objective of the invention is to provide a set of reconstruction allowing a perfect bone consolidation of a fractured tuberosity or two fractured tuberosities with the rest of the humerus, even when the amount of cancellous bone that comprises any such tuberosities is reduced.

The related set of reconstruction includes the features recited in the preamble of the appended claim 1. The term "humeral implant" must be understood as having a very broad sense, referring in particular to a prosthetic stem or an intramedullary nail.

According to the invention, the upper part of the element of reconstruction is constituted by a malleable wall forming a single body with said base part and having a thickness of 0,1 to 1 mm. This sidewall and this base part may in particular be, in this case, in metallic material.

The element of reconstruction according to the invention constitutes generally, by its upper part, a means of filling the external proximal metaphyseal part of the humerus. This upper part allows to form a lateral curved surface placed around a part of the outer periphery of a humeral implant of shoulder prosthesis and being away from the external surface of the humeral implant and at least from one of the anterior or posterior surfaces of this humeral implant. This lateral surface shape has hence a large receiving surface of a fractured tuberosity or of two fractured tuberosities; given the extent of this surface and of its curvature, it allows to perfectly reposition and immobilise any such tuberosities. In addition, this side surface has also as essential function to retain perfectly any such tuberosities in position, by forming a "barrier" opposing any displacement of any such tuberosities towards the inner side of the joint.

The element of reconstruction forms hence a "basket" able of receiving one or several grafts promoting the anchoring of tuberosity or tuberosities by growth of bone cells.

The base part and/or upper part may comprise extension parts, extending, after mounting on the humeral implant along, at least one of the anterior or posterior surfaces of this humeral implant, these base part and/or upper part thus having a "U" or "horseshoe" shape.

Conveniently, said upper part is perforated so that its outer surface communicates with the inner surface thereof.

This upper part delimits, with the wall of the implant, an inner housing able to receive one or more grafts, which allow achieving a perfect bone consolidation of the tuberosity or the tuberosities with the rest of the humerus, the cancellous bone of the tuberosity or the tuberosities being placed in contact with the graft or grafts through the perforations comprised by said upper part. The growth of bone cells may therefore occur between this cancellous bone and this or these grafts, through these perforations. The presence of the graft or the grafts, whether natural or synthetic, allows supplementing, should it be necessary, a small amount of cancellous bone at the tuberosity or tuberosities. In addition, the perforations comprised by said upper part allow the positioning through the latter of threads, cerclages, screws or other means of retaining in position the tuberosity or the tuberosities, this operation being perfectly adapted to the diversity of the shapes that may have tuberosities according to the configuration of the fracture.

Said upper part may comprise a solid wall having a plurality of holes close together; it may also have a grid or mesh structure.

Preferably, said upper part has a rigid structure but able of being deformed manually, being thus malleable. In particular, it may be made in a part of sheet metal of suitable thickness, for example in the order of 0,1 to 1 mm thickness. The shape of said upper part may hence be adapted to the specific forms of the fractured tuberosity or tuberosities, depending on the configuration of the fracture to be treated.

If necessary, particularly when said upper part is intended for the replacement of two tuberosities and that it therefore extends over a large area (about 180°) of the outer periphery of the humeral implant, said upper part comprises at least one slot extending from an upper free edge of this upper part, this slot allowing to split the upper part in two portions at least predominantly independent of one another, able of being deformed independently of one another.

Alternatively or in addition to this possibility of deformation of said upper part, said means for mounting of the element of reconstruction on the humeral implant may have a deformable structure allowing an adaptation of the position of the element of reconstruction in relation to the humeral implant. This adaptation allows the element of reconstruction to be better positioned according to the shape of the fractured tuberosity or tuberosities, which itself results from the configuration of the fracture.

These mounting means could be such as to secure a permanent mounting of the element of reconstruction on the humeral implant, for example by welding of this element on this implant. Preferably, however, these mounting means allow a removable connection of the element of the reconstruction to the humeral implant.

Conveniently, in this case, said mounting means comprise:
- a lug secured to the element of reconstruction, pierced with a hole;
- a threaded hole located on the humeral implant, and
- a screw able to be engaged through the hole of the lug and then be screwed into the hole of the humeral implant.

The humeral implant may in particular comprise a medullar stem, and said hole may be the nearest hole that usually comprises the metaphyseal part of such a medullar stem, allowing the connection of this stem to an introduction and/or impaction instrument in the medullary canal of the humerus.

Said base part is preferably conveniently perforated, which not only increases the deformation capacity thereof, but above all allows promoting bone exchanges through it.

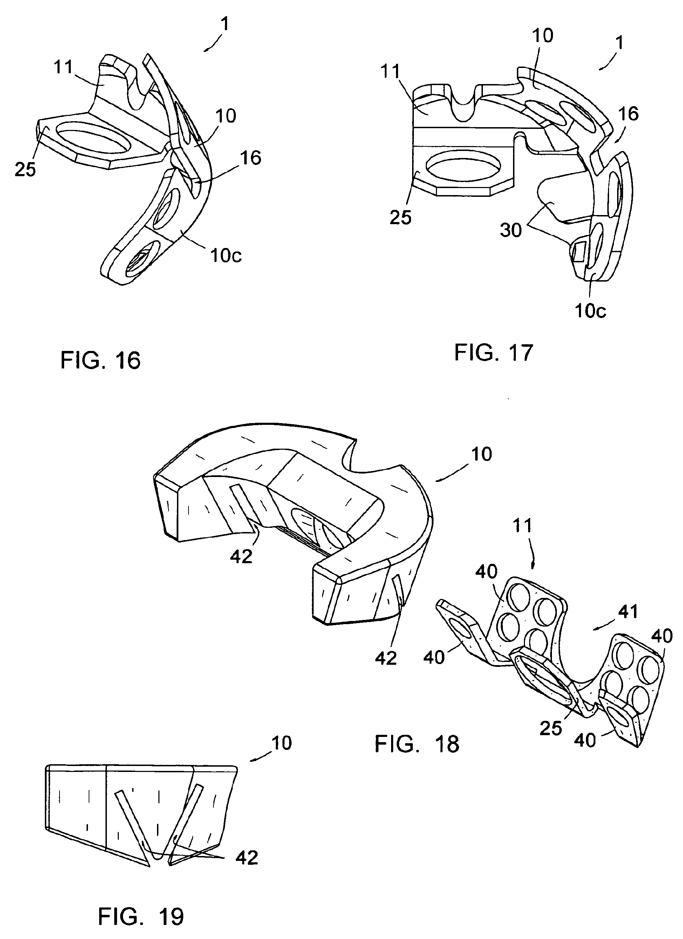

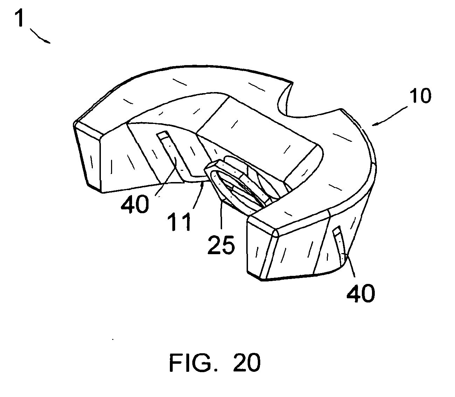

The invention will be better understood, and other characteristics and advantages thereof will become evident, with reference to the attached schematic drawing, representing, as non limiting examples, several possible embodiments of the set of the reconstruction that it relates, wherein Figures 18 to 20 do not show the present invention.
Figure 1 is a perspective view of an element of reconstruction that comprises this set, according to a first embodiment;
Figure 2 is a side view of this element of reconstruction;
Figure 3 is a side view, according to a perpendicular direction in relation to the view direction according to Figure 2;
Figure 4 is a top view;
Figure 5 is a front view of a medullar stem of a shoulder prosthesis equipped with this element of reconstruction;
Figure 6 is a sagittal view of this medullar stem and of this element of reconstruction, of the external side;
Figure 7 is a view of this medullar stem and of this element of reconstruction similar to Figure 5, in section according to a median anteroposterior plane of the medullar stem;
Figure 8 is a view of this medullar stem and of this element of reconstruction substantially according to the axis of a screw for mounting of the element of reconstruction on the medullar stem;
Figure 9 is a front view of the humeral part of a shoulder prosthesis comprising a prosthetic joint head, after placement of this head on a humeral medullar stem;
Figure 10 is a front view of the humeral part of a shoulder prosthesis comprising a plate of reintegration of the native humeral head, after setting up this plate on a humeral medullar stem;
Figure 11 is a view of the humeral part shown in Figure 10 according to the axis of a screw for mounting of the element of reconstruction on the medullar stem;
Figure 12 is a perspective view of the element of reconstruction according to a second embodiment;
Figure 13 is a top view;
Figure 14 is a side view;
Figure 15 is a perspective view of various sizes that this element of reconstruction may have, the elements of reconstruction of different sizes being fictitiously represented as being embedded within each other;
Figure 16 is a perspective view of the element of reconstruction according to a third embodiment;
Figure 17 is a view from above;
Figure 18 which does not show the present invention, is a perspective view of another element of reconstruction, before assembly of a base part and of an upper part that it comprises;
Figure 19 is a side view of this upper part, and
Figure 20 is a perspective view of the element of reconstruction similar to Figure 18, after assembly of the base part and of the upper part.

For simplicity, parts or elements of an embodiment which are found of an identical or similar manner in another embodiment will be identified by the same reference numerals and will not be described again.

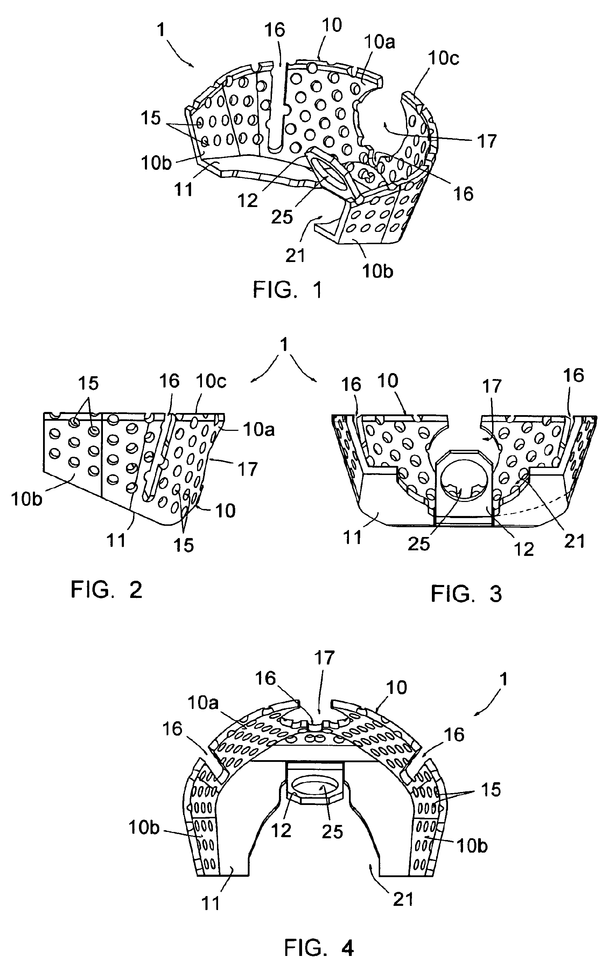

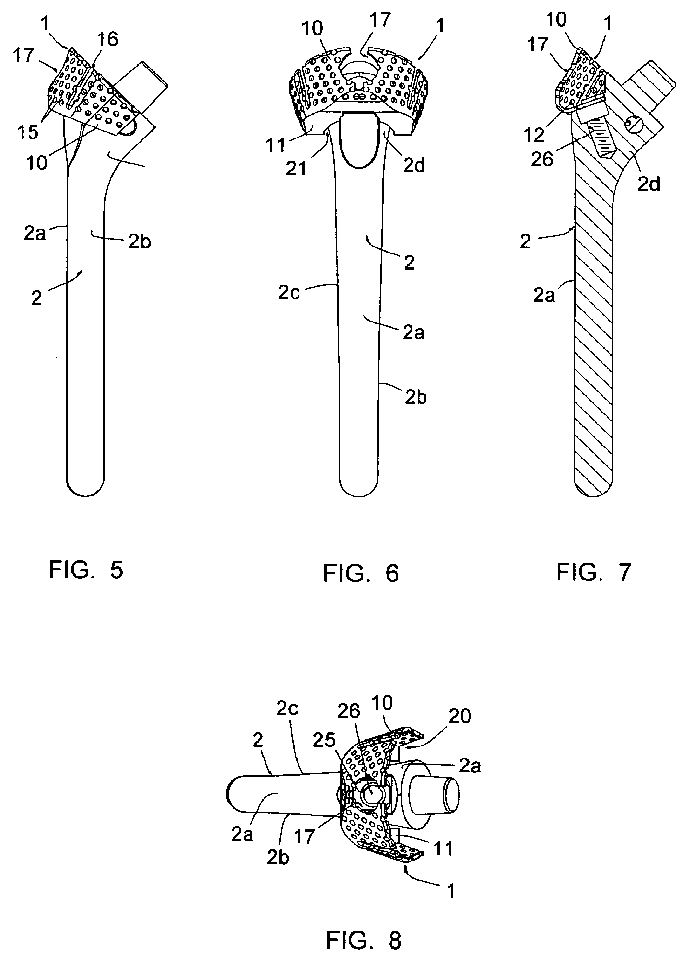

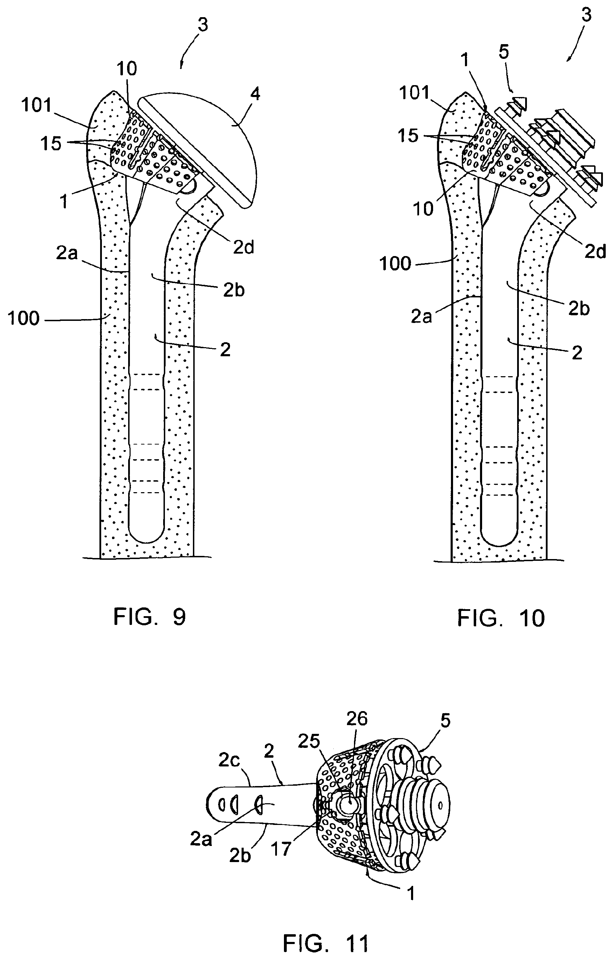

Figures 1 to 4 represent an element 1 of reconstruction of a fractured shoulder joint; Figures 5 to 8 represent a medullary humeral implant 2 in a shoulder prosthesis equipped with this element of reconstruction 1, and figures 9 to 11 represent the humeral part 3 of a shoulder prosthesis, comprising the implant 2 equipped with the element of reconstruction 1 and receiving either a prosthetic head 4 (Figure 9), or a reintegration plate for the reintegration of the native humeral head (Figures 10 and 11).

As known, the implant 2 comprises a proximal end slightly tapered for mounting the head 4 or plate, which comprise corresponding lightly tapered cavities. The implant 2 may comprise transverse locking holes in the humerus (see Figures 9 to 11), or be devoid of such holes (see Figures 5 to 8).

This humeral implant 2 comprises an external surface 2a, an anterior surface 2b and a posterior surface 2c intended to be positioned respectively at the external, anterior and posterior sides of a humerus 100.

With reference to Figures 1 to 4, it is evident that the element of reconstruction 1 according to the first embodiment comprises an upper wall 10, a base wall 11 and a lug 12 allowing the mounting thereof on the implant 2. The set is made of a biocompatible rigid material but able to be deformed manually, in particular in a 1 mm thick pure titanium or alloy sheet, the upper wall 10 being integral with the base wall 11.

The upper wall 10 extends along a curved outer edge of the base wall 11. It is particularly evident in Figure 4 that it has a main part 10a of a generally rounded shape, extending to about 180 degrees, extended at its ends by two plane walls 10b converging towards one another in this embodiment. It is pierced with several holes 15 that pass through it from side to side, giving it a perforated structure.

The upper wall 10 has three slots 16 extending from its free edge 10c opposite the base wall 11 up to near this base wall, and which are evenly spaced around the circumference of this wall 10 so that there is a median slot and two lateral slots. These slots 16 allow dividing the upper wall 10 in three wall sections independent of each other, able to be deformed manually independently of each other.

In addition, the wall 10 has, at the centre slot 16, a circular opening 17 allowing to provide access from the exterior wall 10, to an opening 25 that comprises the lug 12 (see Figures 3 and 8).

The base wall 11 delimits with the wall 10 a housing 20 thus making the element of reconstruction 1 to have the shape of a basket. It may be solid as shown in the example, or may be perforated along the lines of the wall 10. It has two side parts delimiting between them a broad notch 21 which allows its engagement around the metaphyseal part 2d of the implant 2 (see Figures 5 to 8), thus making the element to have a "horseshoe" shape. The notch 21 is shaped and sized so that the edge which delimits it lies close to the wall of the metaphyseal part 2d of the implant 2 when the element of reconstruction 1 is positioned on this implant 2, said side parts of the base wall 11 extending along the front 2b and rear 2c surfaces of the humeral implant 2.

The lug 12 is formed by a median part of the sheet constituting the base wall 11, this sheet being cut on three sides according to a general shape more or less rectangular. The lug 12 remains connected to the base wall 11 by the fourth side, parallel to an imaginary line connecting the two side ends of the central part 10a of the wall 10, and is folded at the fourth side so as to have an angulation in the order of about fifty degrees in relation to the plane comprising the free edge 10c of the wall 10 (see Figure 2). The width of the lug 12 remains relatively small, not exceeding one third of the total width of the base wall 11 (see Figure 4), so that this lug 12 has a manually deformable structure, allowing an adaptation of the position of the element of reconstruction 1 in relation to implant 2.

The lug 12 comprises an opening 25 passing it through from side to side. It is positioned so that in the mounting position on the implant 2 this opening 25 is in coincidence with a threaded and rolled proximal hole 26 (see Figure 7) that comprises the metaphyseal part 2d, this hole being generally provided on such a metaphyseal part to enable connection of the implant 2 to an introduction and/or impaction instrument of this implant in the medullary canal of the humerus.

In practice, as it is evident with reference to Figures 5 to 11, the implant 2 is inserted into the medullary canal of a humerus 100 (see Figure 9 or 10) of which the metaphyseal part has previously been resected accordingly.

The element of reconstruction 1 is then engaged around the metaphyseal part 2d of the implant 2, on the external side thereof, through the notch 21 that comprises the base wall 11, up to the opening 25 of the lug 12 in coincidence of the hole 26, then a screw is inserted through the opening 17 and is positioned through the opening 25 and in this hole 26 to ensure mounting of the element of reconstruction 1 on the implant 2. This screw is operated by using a screwdriver (not shown) that may be engaged through the wall 10 through opening 17 (see Figure 8 and 11).

In this mounting position, the base wall 11 extends beyond the external surface 2a of the implant 2 and projects beyond the front 2b and rear 2c surfaces of this implant. The outer curved edge of the wall 11, and therefore the upper wall 10, extends accordingly away from these surfaces 2a, 2b and 2c, as shown in Figures 5 to 8.

It is evident from figure 9 to 11 that the element of reconstruction 1 constitutes globally, by its base wall 11 and its upper wall 10, a means of bridging the proximal outer area of the metaphyseal part of the humerus. The wall 10 enables to form a curved external side surface positioned around a part of the outer periphery of the humeral implant 2 and located away from the external 2a, front 2b and rear 2c surfaces of this implant, which forms a broad receiving surface of a fractured tuberosity 101 or of two fractured tuberosities 101; given the extent of this surface and its curvature, it enables to reposition and immobilise perfectly any such tuberosities 101. In addition, the essential function of the upper part 10 is also to retain perfectly any such tuberosities 101 in position, forming a "barrier" opposing any displacement of the tuberosity or tuberosities 101 towards the inner side of the joint.

In addition, the housing 20 substantially in a "U" shape located between the metaphyseal part 2d and the upper wall 10 is able to receive one or more natural or synthetic grafts. The prosthetic humeral head 4 or the plate is then positioned, allowing the closing of this housing 20 and thus confining the graft or grafts contained therein.

The head 4 has a spherical cap shape.

The plate, in turn, comprises a base plate, a central stud, retentive peripheral studs and openings passing through the base plate (see Figure 11).

The fractured tuberosities 101 may be fixed to the wall 10 by means of passing threads through holes 15. The graft or grafts located within the housing 20 and the repositioning of the tuberosities 101 on the external surface of the upper wall 10 are able to achieve a perfect bone consolidation of the tuberosities 101 with the rest of the humerus 100, the cancellous bone of the tuberosity or tuberosities being placed in contact with one or more grafts through the perforations comprised by the upper wall 10 and the growth of the bone cells between this cancellous bone and this graft, taking place through these perforations. The presence of this or these natural or synthetic grafts, allows supplementing, should it be necessary, a small amount of cancellous bone at the tuberosities 101.

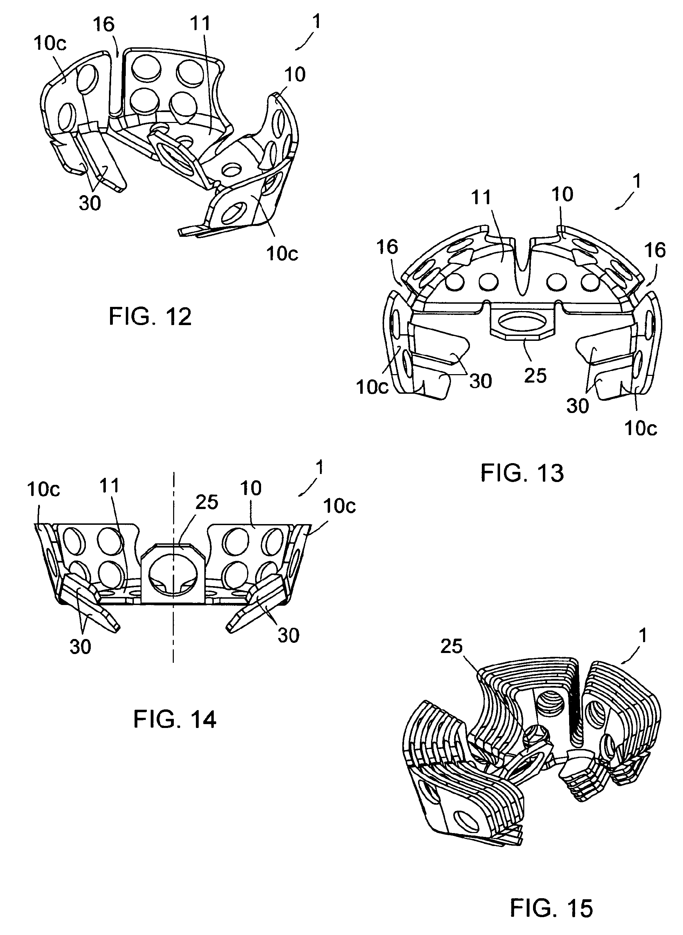

With reference to Figures 12 to 14, it is evident that the element of reconstruction 1 according to a second embodiment comprises a base wall 11 not forming parts intended to extend along the front 2b and rear 2c surfaces of the implant 2; the wall 10 is extended beyond the inner edge of the wall 11 by two extension walls 10c intended to extend along these surfaces 2b and 2c. A slot 16 is provided between the wall 10 and each wall 10c, causing the latter to be connected to the rest of the element 1 by a relatively reduced part of the section, easily deformable. To stabilise the walls 10c and close the housing 20 formed by element 1 beyond the wall 11, each wall 10c comprises two lower fins 30.

Figure 15 shows that the set of reconstruction according to the invention may comprise a series of elements of a reconstruction 1 of different sizes. These elements of reconstruction 1 are fictitiously represented embedded into each other in this Figure 15, the lugs 25 of the various elements 1 normally forming an obstacle to such an embedding.

Figures 16 and 17 show that the element of reconstruction 1 according to the third embodiment is similar to the element 1 according to the second embodiment, with a base wall 11 interrupted and an extending wall 10c of wall 10. In this case, the element 1 of reconstruction is planned for the replacement of only one of the tuberosities 101, and to extend on the side of only one front 2b or rear 2c wall.

With reference to Figures 18 to 20, it is evident that another element of reconstruction 1 comprises an upper part 10 formed by a separate element of a base part 11 and is connected thereto by assembly.

The base part 11 is a metallic material and comprises lugs 40 arranged in a "V" shape, pierced by holes, which are located on either side of a notch 41 allowing the insertion of the screw and the lug 25.

The upper part 10 is formed by a block of porous materials, whether natural or synthetic, adapted to the growth of bone cells. It has respectively cleavers 42, intended for receiving the lugs 40 by adjusted engagement. This part 10 forms the previously referred curved external surface and has, in the shown example, the "horseshoe" shape, also previously referred.

It is evident from the foregoing that the invention provides a set of reconstruction allowing achieving the following objectives:
- allow the repositioning of the tuberosities 101 so that bone consolidation takes place under optimum conditions;
- allow preventing effectively the risk of displacement of the tuberosity or tuberosities 101 towards the inner side of the joint;
- possibility to adjust the position of the element of reconstruction 1 in relation to the humeral implant 2 depending on the configuration of the fracture;
- obtaining a perfect bone consolidation of the fractured tuberosities with the rest of the humerus, even when the amount of cancellous bone that comprises these tuberosities is reduced.

The invention has been described above with reference to embodiments given by way of an example. It is understood that it is not limited to these embodiments but that it extends to all other embodiments covered by the hereby annexed claims.

## Claims

1. Set of reconstruction of a fractured shoulder joint, comprising a humeral implant (2) and an element of reconstruction (1) intended to be connected to this humeral implant (2); the humeral implant (2) comprises an external surface (2a), an anterior surface (2b) and a posterior surface (2c) intended to be placed respectively at the external, anterior and posterior sides of a humerus (100), and the element of reconstruction (1) is, after connection to the humeral implant (2), located on the external side of this humeral implant (2) and is able of receiving one fractured tuberosity (101) or the two fractured tuberosities; the set of reconstruction comprises at least one element of reconstruction (1) comprising a base part (11) and an upper part (10);
- said base part (11) comprises means (12, 25, 26) for its mounting on the external side of the humeral implant (2); it is sized to project, after installation on the humeral implant (2), beyond the external surface (2a) of the humeral implant (2) and beyond at least one of the anterior (2b) or posterior (2c) surfaces of this humeral implant (2), on the outer side of these surfaces; and
- said upper part (10) forms an outer external curved surface extending, after mounting on the humeral implant (2), away from the external surface (2a) of this humeral implant (2) and from at least one of the anterior (2b) or posterior (2c) surfaces of this humeral implant (2);
**characterized in that** said upper part (10) is constituted by a malleable wall forming a single body with said base part (11) and having a thickness of 0,1 to 1 mm.

2. Set according to claim 1, **characterized in that** the base part (11) and/or upper part (10) comprise extension parts, extending, after mounting on the humeral implant (2) along, at least one of the anterior (2b) or posterior (2c) surfaces of this humeral implant (2), these base part (11) and/or upper part (10) thus having a "U" or "horseshoe" shape.

3. Set according to claim 1 or claim 2, **characterized in that** said upper part (10) is perforated so that its outer surface communicates with the inner surface thereof.

4. Set according to claim 3, **characterized in that** said upper part (10) has a grid or mesh structure.

5. Set according to anyone of claims 1-4, **characterized in that** said upper part (10) has a rigid structure but able of being deformed manually, being thus malleable.

6. Set according to anyone of claims 1-5, **characterized in that** said upper part (10) comprises at least one slot (16) extending from an upper free edge (10c) of this upper part (10), this slot (16) allowing to split the upper part (10) in two portions at least predominantly independent of one another, able of being deformed independently of one another.

7. Set according to anyone of claims 1-6, **characterized in that** said means (12, 25, 26) for mounting of the element of reconstruction (1) on the humeral implant (2) have a deformable structure allowing an adaptation of the position of the element of reconstruction (1) in relation to the humeral implant (2).

8. Set according to anyone of claims 1-7, **characterized in that** said mounting means comprise:
- a lug (12) secured to the element of reconstruction (1), pierced with a hole (25);
- a threaded hole (26) located on the humeral implant (2), and
- a screw able to be engaged through the hole (25) of the lug (12) and then be screwed into the hole (26) of the humeral implant (2).

9. Set according to anyone of claims 1-8, **characterized in that** it comprises a prosthetic humeral head (4) separated from the humeral implant (2).

10. Set according to anyone of claims 1-9, **characterized in that** it comprises a reintegration plate for the reintegration of the native humeral head, separated from the humeral implant (2), this reintegration plate comprising a base plate, a central stud, retentive peripheral studs and openings passing through the base plate.

11. Set according to anyone of claims 1-10, **characterized in that** it comprises a series of elements of reconstruction (1) of different sizes.

## Patentansprüche

1. Rekonstruktionssatz eines gebrochenen Schultergelenks, aufweisend ein Humerusimplantat (2) und ein Rekonstruktionselement (1), das vorgesehen ist mit diesem Humerusimplantat verbunden zu werden (2); das Humerusimplantat (2) weist eine externe Fläche (2a), eine anteriore Fläche (2b) und eine posteriore Fläche (2c) auf, die vorgesehen sind an der externen, anterioren beziehungsweise posterioren Seite eines Humerus (100) angeordnet zu werden, und das Rekonstruktionselement (1) ist, nach Verbindung zu dem Humerusimplantat (2), an der externen Seite dieses Humerusimplantats (2) angeordnet und kann einen gebrochenen Tuberositas (101) oder zwei gebrochene Tuberositae aufnehmen; der Rekonstruktionssatz weist mindestens ein Rekonstruktionselement (1) auf, das ein Basisteil (11) und ein Oberteil (10) aufweist;
- das Basisteil (11) weist ein Mittel (12, 25, 26) zu seinem Befestigen an der externen Seite des Humerusimplantats (2) auf; das Basisteil (11) hat eine derartige Größe, dass es nach Anbringung an dem Humerusimplantat (2) über die externe Fläche (2a) des Humerusimplantats (2) hinausragt und über mindestens eine der anterioren (2b) oder posterioren (2c) Flächen dieses Humerusimplantats (2) an der Außenseite dieser Flächen hinausragt; und
- das Oberteil (10) bildet eine äußere externe gekrümmte Fläche, die sich nach Befestigen an dem Humerusimplantat (2) weg von der externen Fläche (2a) dieses Humerusimplantats (2) und von mindestens einem der anterioren (2b) oder posterioren (2c) Flächen dieses Humerusimplantats (2) erstreckt;
**dadurch gekennzeichnet, dass** das Oberteil (10) gebildet ist durch eine verformbare Wand, die einen einzelnen Körper mit diesem Basisteil (11) bildet und eine Dicke von 0,1 bis 1 mm hat.

2. Satz gemäß Anspruch 1, **dadurch gekennzeichnet, dass** das Basisteil (11) und/oder Oberteil (10) Erstreckungsteile aufweist, die sich nach Befestigen an dem Humerusimplantat (2) entlang mindestens einer der anterioren (2b) oder posterioren (2c) Flächen dieses Humerus-implantats (2) erstrecken, dieses Basisteil (11) und/oder Oberteil (10) hat dadurch eine "U"- oder "Hufeisen"-form.

3. Satz gemäß Anspruch 1 oder 2, **dadurch gekennzeichnet, dass** das Oberteil (10) perforiert ist, so das seine äußere Fläche mit seiner inneren Fläche kommuniziert.

4. Satz gemäß Anspruch 3, **dadurch gekennzeichnet, dass** das Oberteil (10) eine Gitter- oder eine Netzstruktur hat.

5. Satz gemäß einem der Ansprüche 1-4, **dadurch gekennzeichnet, dass** das Oberteil (10) eine starre Struktur hat, die aber manuell deformierbar ist, daher verformbar ist.

6. Satz gemäß einem der Ansprüche 1-5, **dadurch gekennzeichnet, dass** das Oberteil (10) mindestens einen Schlitz (16) aufweist, der sich von einer oberen freien Kante (10c) diese Oberteils (10) erstreckt, dieser Schlitz (16) erlaubt es das Oberteil (10) in zwei Teile zu teilen, die überwiegend unabhängig voneinander sind, unabhängig voneinander deformierbar sind.

7. Satz gemäß einem der Ansprüche 1-6, **dadurch gekennzeichnet, dass** das Mittel (12, 25, 26) zum Befestigen des Rekonstruktionselements (1) an dem Humerusimplantat (2) eine deformierbare Struktur hat, die eine Anpassung der Position des Rekonstruktionselements (1) bezüglich des Humerusimplantats (2) erlaubt.

8. Satz gemäß einem der Ansprüche 1-7, **dadurch gekennzeichnet, dass** das Befestigungsmittel aufweist:
- eine Öse (12), die an dem Rekonstruktionselement (1) befestigt ist, von einem Loch (25) durchlocht ist;
- ein Loch (26) mit einem Gewinde, wobei das Loch (26) an dem Humerusimplantat (2) angeordnet ist, und
- eine Schraube, die durch das Loch (25) der Öse (12) eingreifen kann und dann in das Loch (26) des Humerusimplantats (2) geschraubt werden kann.

9. Satz gemäß einem der Ansprüche 1-8, **dadurch gekennzeichnet, dass** er einen prothetischen Humeruskopf (4) aufweist, der von dem Humerusimplantat (2) getrennt ist.

10. Satz gemäß einem der Ansprüche 1-9, **dadurch gekennzeichnet, dass** er eine Reintegrationsplatte zum Reintegrieren des natürlichen Humeruskopfs aufweist, wobei die Reintegrationsplatte von dem Humerusimplantat (2) getrennt ist, diese Reintegrationsplatte weist eine Basisplatte, einen zentralen Stift, haltende Umfangsstifte und Öffnungen auf, die durch die Basisplatte hindurchgehen.

11. Satz gemäß einem der Ansprüche 1-10, **dadurch gekennzeichnet, dass** er eine Serie von Rekonstruktionselementen (1) unterschiedlicher Größe aufweist.

## Revendications

1. Ensemble de reconstruction d'une articulation de l'épaule fracturée, comprenant un implant huméral (2) et un élément de reconstruction (1) destiné à être relié à cet implant huméral (2) ; l'implant huméral (2) comprend une face externe (2a), une face antérieure (2b) et une face postérieure (2c) destinées à être placées respectivement au niveau des côtés externe, antérieur et postérieur d'un humérus (100), et l'élément de reconstruction (1) se trouve, après liaison à l'implant huméral (2), situé sur le côté externe de cet implant huméral (2) et est apte à recevoir une tubérosité (101) fracturée ou les deux tubérosités fracturées ; l'ensemble de reconstruction inclut au moins un élément de reconstruction (1) comprenant une partie de base (11) et une partie supérieure (10) ;
- ladite partie de base (11) comprend des moyens (12, 25, 26) pour son montage sur le côté externe de l'implant huméral (2) ; elle est dimensionnée de manière à faire saillie, après montage sur l'implant huméral (2), au-delà de la face externe (2a) de l'implant huméral (2) et au-delà d'au moins une des faces antérieure (2b) ou postérieure (2c) de cet implant huméral, sur le côté extérieur de ces faces, et
- ladite partie supérieure (10) forme une face extérieure courbe s'étendant, après montage sur l'implant huméral (2), à distance de la face externe (2a) de cet implant huméral et d'au moins une des faces antérieure (2b) ou postérieure (2c) de cet implant huméral (2) ;
**caractérisé en ce que** cette partie supérieure (10) est constituée par une paroi malléable formant corps avec ladite partie de base (11) et ayant une épaisseur de 0,1 à 1 mm.

2. Ensemble selon la revendication 1, **caractérisé en ce que** la partie de base (11) et/ou la partie supérieure(10) comprend des parties d'extension, s'étendant, après montage sur l'implant huméral (2), le long d'au moins une des surfaces antérieure (2b) ou postérieure (2c) de cet implant huméral (2), ces parties de base (11) et/ou partie supérieure ayant ainsi une forme en "U" ou en "fer à cheval".

3. Ensemble selon la revendication 1 ou la revendication 2, **caractérisé en ce que** ladite partie supérieure (10) est ajourée de telle sorte que sa face externe communique avec sa face interne.

4. Ensemble selon la revendication 3, **caractérisé en ce que** ladite partie supérieure (10) a une structure en grille ou grillage.

5. Ensemble selon l'une des revendications 1 à 4, **caractérisé en ce que** ladite partie supérieure (10) a une structure rigide mais apte à être déformée manuellement, étant ainsi malléable.

6. Ensemble selon l'une des revendications 1 à 5, **caractérisé en ce que** ladite partie supérieure (10) comprend au moins une saignée (16) s'étendant depuis un bord libre supérieur (10c) de cette partie supérieure (10), cette saignée (16) permettant de diviser la partie supérieure (10) en deux portions au moins majoritairement indépendantes l'une de l'autre, aptes à être déformées indépendamment l'une de l'autre.

7. Ensemble selon l'une des revendications 1 à 6, **caractérisé en ce que** lesdits moyens de montage (12, 25, 26) de l'élément de reconstruction (1) à l'implant huméral (2) présentent une structure déformable permettant une adaptation de la position de l'élément de reconstruction (1) par rapport à l'implant huméral (2).

8. Ensemble selon l'une des revendications 1 à 7, **caractérisé en ce que** lesdits moyens de montage comprennent :
- une patte (12) solidaire de l'élément de reconstruction (1), percée d'un trou (25) ;
- un trou taraudé (26) aménagé sur l'implant huméral (2), et
- une vis apte à être engagée au travers du trou (25) de la patte (12) puis à être vissée dans le trou taraudé (26) de l'implant huméral (2).

9. Ensemble selon l'une des revendications 1 à 8, **caractérisé en ce qu'**il comprend une tête humérale prothétique (4) séparée de l'implant huméral (2).

10. Ensemble selon l'une des revendications 1 à 9, **caractérisé en ce qu'**il comprend une platine de réinsertion de la tête humérale native, séparée de l'implant huméral (2), cette platine de réinsertion (5) incluant une plaque d'embase, un plot central et des plots périphériques rétentifs, et des ouvertures traversant la plaque d'embase.

11. Ensemble selon l'une des revendications 1 à 10, **caractérisé en ce qu'**il comprend une série d'éléments de reconstruction (1) de différentes tailles.
